(19) Europäisches Patentamt
European Patent Office
Office européen des brevets

(11) Publication number: **0 350 293 B1**

(12) **EUROPEAN PATENT SPECIFICATION**

(45) Date of publication of patent specification: **11.01.95** (51) Int. Cl.$^{6}$: **C07H 19/073**

(21) Application number: **89306835.3**

(22) Date of filing: **05.07.89**

(54) **Process for preparing derivatives of 2'-deoxy-beta-cytidine and salts thereof.**

(30) Priority: **05.07.88 JP 166062/88**

(43) Date of publication of application:
**10.01.90 Bulletin 90/02**

(45) Publication of the grant of the patent:
**11.01.95 Bulletin 95/02**

(84) Designated Contracting States:
**DE FR GB**

(56) References cited:
**EP-A- 0 175 004**
**EP-A- 0 300 765**
**DE-A- 1 919 307**
**GB-A- 1 601 020**
**US-A- 3 868 373**

(73) Proprietor: **Japan Tobacco Inc.**
**2-1 Toranomon, 2-Chome**
**Minato-Ku**
**Tokyo 105 (JP)**

Proprietor: **YUKI GOSEI KOGYO CO., LTD.**
**3-24, Hirakawa-cho 2-chome**
**Chiyoda-ku**
**Tokyo 102 (JP)**

(72) Inventor: **Kawakami, Hiroshi Life Science Research Laboratory**
**Japan Tobacco Inc**
**6-2 Umegaoka**
**Midori-ku**
**Yokohama-shi**
**Kanagawa 227 (JP)**
Inventor: **Matsushita, Hajime Life Science Research Lab.**
**Japan Tobacco Inc**
**6-2 Umegaoka**
**Midori-ku**
**Yokohama-shi**
**Kanagawa 227 (JP)**
Inventor: **Mizutani, Nobuhiro c/o Tokyo Laboratory**
**Yuki Gosei Kogyo Co.,Ltd.**
**37-1 Sakashita 3-chome**
**Itabashi-ku**
**Tokyo 174 (JP)**
Inventor: **Takiguchi, Toshimi c/o Tokyo Laboratory**
**Yuki Gosei Kogyo Co.,Ltd.**
**37-1 Sakashita 3-chome**
**Itabashi-ku**
**Tokyo 174 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Itoh, Kazuo c/o Tokyo Laboratory**
**Yuki Gosei Kogyo Co.,Ltd.**
**37-1 Sakashita 3-chome**
**Itabashi-ku**
**Tokyo 174 (JP)**
Inventor: **Naoi, Yoshitake c/o Tokyo Laboratory**
**Yuki Gosei Kogyo Co.,Ltd.**
**37-1 Sakashita 3-chome**
**Itabashi-ku**
**Tokyo 174 (JP)**

(74) Representative: **Holmes, Michael John et al**
**Frank B. Dehn & Co.**
**European Patent Attorneys**
**Imperial House**
**15-19 Kingsway**
**London, WC2B 6UZ, (GB)**

## Description

The present invention relates to a process for preparing derivatives of 2′deoxy-β-cytidine having the following general formula (I) and salts thereof:

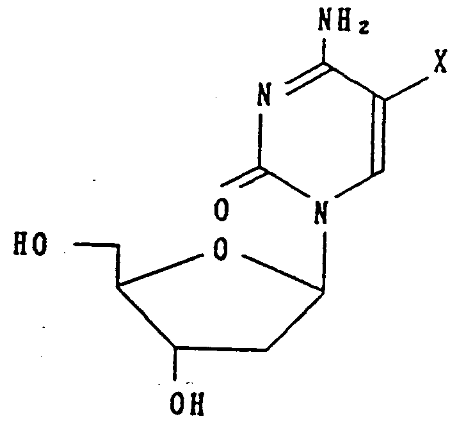

( I )

(wherein X is a hydrogen atom, a halogen atom, or an alkyl group or alkenyl group which may be substituted with one or more halogen atoms).

Hereinafter, "derivatives of 2′-deoxy-β-cytidine" are referred to as "β-cytidine derivative (I)."

β-cytidine derivative (I) is a medically useful compound, which is antiviral or antumorigenic, and a useful raw material for preparing 2′,3′-dideoxycytidine which is an effective remedy for AIDS (Acquired Immune Deficiency syndrome).

In the conventional process for preparing β-cytidine derivative (I), 2′-deoxy-β-cytidine is made by decomposing natural DNA (deoxyribonucleic acid) with enzymes.

In the chemical synthetic methods, $0^{3,5}$-bis(4-chlorobenzoyl)-2-deoxy-D-ribofuranosyl chloride is allowed to condense with an N-acylcytosine mercury salt to obtain a mixture of α- and β-isomers (1:1) and after the β-isomer is separated, the protecting groups are eliminated to yield 2'-deoxy-β-cytidine [Journal of the American Chemical Society, 83, 4066(1961)]. Alternatively, 2'-deoxy-β-cytidine is prepared by eliminating the hydroxy group in the 2'-position of cytidine [Japanese TOKKYO-KOKAI-KOHO (18-month Publication of Unexamined Patent Application) SHOWA 57(1982)-16482 and Journal of the American Chemical Society, 105, 4059(1983)].

In the conventional process for preparing the above β-cytidine derivative [I], the process of decomposing natural deoxyribonucleic acid is not suitable for industrial production because access to the starting material is restricted.

In the first mentioned chemical synthesis, there is the disadvantage that mercury used is poisonous, and only a mixture of α-isomer and β-isomer in the ratio of 1:1 is obtained.

In the second chemical synthesis, there is the disadvantage that hydrogen which is dangerous to handle must be employed, expensive catalysts are required and further poisonous tin compounds are used. Therefore, these processes are not industrially suitable.

Further examples of the chemical synthesis of 2'-deoxy-β-cytidine are found in EP-A-175004, GB 1601020, US 3868373 and DE-A-1919307. However all suffer from the same disadvantage as the first chemical synthesis mentioned above, namely production of a 1:1 mixture of α and β-isomers.

The inventors found that β-cytidine derivatives and salts thereof can be prepared with a high selectivity of the β-isomer and in a high yield by the method shown below.

Further, in the case of need, physiologically allowable salts of the β-cytidine derivative can be prepared.

The present invention provides a process for preparing derivatives of 2′-deoxy-β-cytidine having the following general formula [I] and salts thereof

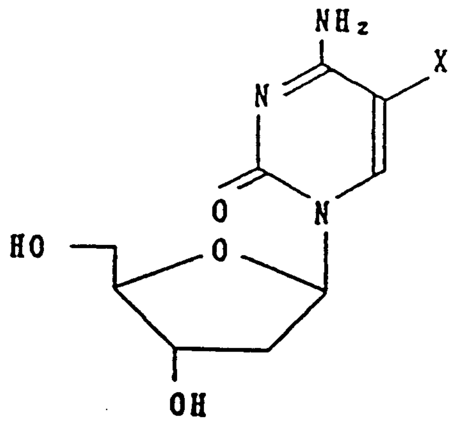

( I )

(wherein X is a hydrogen atom, a halogen atom, or an alkyl or alkenyl group which may be substituted with one or more halogen atoms)
characterized in that a derivative of 1-α-halogeno-2-deoxyribose having the following general formula [III]:

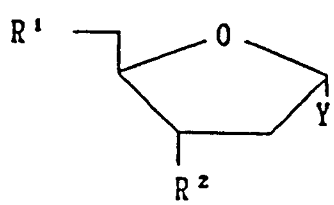

( III )

(wherein $R^1$ and $R^2$ are independently protected hydroxyl groups and Y is a halogen atom)
is allowed to condense with a derivative of cytosine having the following general formula [IV]:

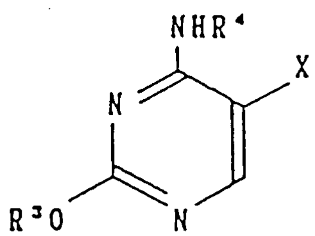

( IV )

(wherein $R^3$ is hydroxyl protecting group, $R^4$ is an amine protecting group and X is as defined above) in the presence of ammonia or an amine using 1.1 to 1.5 equivalents of cytosine derivative (IV) per equivalent of ribose derivative (III) and 0.5 to 1.5 equivalents of ammonia or amine per equivalent of cytosine derivative (IV) or in the absence of ammonia or an amine using 3 to 5 equivalents of cytidine derivative (IV) per equivalent of ribose derivative (III) to obtain a derivative of 3',5'-di-substituted-2'-deoxy-β-cytidine having the following general formula [II]:

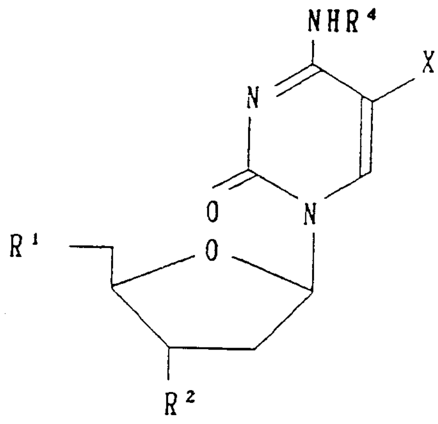

( II )

(wherein $R^1$, $R^2$, $R^4$ and X are as above defined) and then the protecting groups are eliminated.

Groups $R^1$ and $R^2$ of ribose derivative [III] are protected hydroxyl groups in which the protecting groups may be those used as the protecting group for saccharides.

Preferred protecting groups are an aralkyl group such as a benzyl group or a trityl group; an acyl group such as an acetyl group, a propionyl group, a pivaloyl group or a benzoyl group; an alkoxycarbonyl group such as an ethoxycarbonyl group; or an aryloxycarbonyl group such as a phenoxycarbonyl group. Other protecting groups capable of easy elimination may be employed as the protecting group.

When the protecting groups contain a phenyl group, the phenyl group may have a halogen atom, an alkyl group, a nitro group, an alkoxyl group and the like as a sustitutent.

Y is a halogen atom such as a chlorine atom, a bromine atom or an iodine atom.

Groups $R^3$ and $R^4$ of cytosine derivative [IV] are an amine protecting group and a hydroxyl protecting group, and each may independently be an aralkyl group such as a benzyl group or a trityl group; an acyl group such as an acetyl group, a propionyl group, a pivaloyl group or a benzoyl group; an alkoxycarbonyl group such as an ethoxycarbonyl group; an aryloxycarbonyl group such as a phenoxycarbonyl group, or a triorganosilyl group such as a trimethylsilyl group, a t-butyldimethylsilyl group or a phenyldimethylsilyl group. These protecting groups are not limited to the above-mentioned groups.

X is a hydrogen atom, a halogen atom such as a chlorine atom, a bromine atom or an iodine atom, or an alkyl group or alkenyl group which may be substituted with one or more halogen atoms and preferably contains 1-6 carbon atoms.

Aprotic solvents shown below may be used as solvents in the reaction:

Ethers such as diethyl ether, diisopropyl ether and tetrahydrofuran; hydrocarbons such as n-pentane, n-hexane, cyclohexane, benzene and toluene; halogen-containing hydrocarbons such as dichloromethane chloroform and dichloroethane; and acetonitrile.

Most preferred solvents are chloroform, 1,2-dichloroethane and acetonitrile.

Such condensation of ribose derivative [III] with cytosine derivative [IV] is carried out in the aprotic solvent without catalysts.

Selectivity of production of the $\beta$-isomer is increased by using 3 to 5 equivalents of cytosine derivative [IV] per equivalent of ribose derivative [III]: substituted-$\beta$-cytidine derivative [II] can be obtained with a selectivity of 90% or more.

When cytosine derivative [IV] is used in an amount of less than two equivalents per equivalent of ribose derivative [III], the production of $\alpha$-isomer is increased and the selective production of $\beta$-isomer is decreased (unless ammonia or an amine is present). This is not desirable.

The reaction temperature of the condensation depends on the solvent used, and the condensation is carried out at temperatures from 0 °C to 100 °C, preferably 0 °C to 50 °C.

Reaction time depends on the solvents used and/or the reaction temperature, and is generally 30 minutes to 20 hours.

Condensation of ribose derivative [III] with cytosine derivative [IV] may also be carried out in an aprotic solvent in the presence of an amine or ammonia.

Amines having the following general formula [V] may be used:

$R^5{}_nNH_{(3-n)}$ [V]

wherein $R^5$ is an alkyl group such as a methyl group, an ethyl group and a butyl group, a triorganosilyl

group such as a trimethylsilyl, and n is an integer of 1 to 3. The preferred catalysts are, for example, ammonia, triethylamine, hexamethyldisilazane and the like.

When ammonia or an amine is present, cytosine derivative [IV] is used in an amount of 1.1 to 1.5 equivalents, relative to ribose derivative [III] and the ammonia or amine are used in an amount of 0.5 to 1.5 equivalents, relative to cytosine derivative [IV] to increase greatly the selective production of the $\beta$-type compound. It is possible to obtain substituted-$\beta$-cytidine derivative [II] with a selectivity of 90% or more of $\beta$-type compound.

The reaction temperature of the condensation depends on solvents and amines used in the reaction, and the preferred temperature is 0°C to 100°C, preferably 0°C to 50°C. Reaction time depends on the reaction temperatures, and solvents and amines used in the reaction, and the reaction time is preferably 30 minutes to 20 hours.

Substituted-$\beta$-cytidine derivative [II] (including the $\alpha$-isomer in some quantity) is obtained in the condensation, and $\beta$-cytidine derivative [I] can be obtained in a high yield of 75% of more relative to ribose derivative [III] by recrystallizing substituted-$\beta$-cytidine derivative [II] (including $\alpha$-isomer) to obtain substituted-$\beta$-cytidine derivative [II], including the $\beta$-isomer only and then eliminating the protection group. Further salts of $\beta$-cytidine derivative [I] can be prepared, which are physiologically allowable.

Salts of $\beta$-cytidine derivative [I] may be prepared by using inorganic acids such as hydrochloric acid, hydrobromic acid, phosphoric acid and sulfuric acid, and organic acids such as oxalic acid, maleic acid, fumaric acid, lactic acid, tartaric acid, malic acid, citric acid and benzoic acid.

It can be seen from the foregoing that $\beta$-cytidine derivative [I] can be prepared with high selectivity of the $\beta$-isomer and in high yield without using expensive catalysts and dangerous chemicals.

The present invention will be illustrated by effective examples and comparative examples in the following, but the present invention is not restricted thereto.

Example 1

25.5g (100 mmol) of bis(trimethylsilyl)cytosine and 300 ml of chloroform were introduced into a flask (one litre, three necks flask replaced with nitrogen gas), and to the mixture was added 8.59 g (20 mmol) of $0^{3,5}$-bis(4-chlorobenzoyl)-2-deoxy-$\alpha$-D-ribofuranosyl chloride dissolved in 300 ml of chloroform, while stirring, to subject to condensation at room temperature for one hour.

After the reaction was completed, excess cytosine was separated by adding 200 ml of water, and the separated organic layer was condensed to obtain 10.5 g of $0^{3',5'}$-bis(4-chlorobenzoyl)-2′-deoxycytidine.

By analyzing this compound with high performance liquid chromatography (reverse phase column, eluent of 0.05M $NaH_2PO_4$,UV 270 nm; In the following examples, analysis was carried out under the same conditions), the ratio of $\alpha$-type compound to $\beta$-type compound of 9/91 was obtained.

By recrystallizing the crude product from ethanol, 8.57 g of $0^{3',5'}$-bis(4-chlorobenzoyl)-2′-deoxy-$\beta$-cytidine, not contain $\alpha$-type compound, was obtained. Yield 85 % [to $0^{3,5}$-bis(4-chlorobenzoyl)-2-deoxy-$\alpha$-D-ribofuranosyl chloride].

$0^{3',5'}$-bis(4-chlorobenzoyl)-2'-deoxy-$\beta$-cytidine was treated with ammonia-containing methanol to eliminate the protecting group (4-chlorobenzoyl group) and then concentrated hydrochloric acid was added to produce crude 2′-deoxy-$\beta$-cytidine HCl, and the crude salt was recrystallized from water-methanol to obtain 4.32 g of 2′-deoxy-$\beta$-cytidine HCl. Yield 82 % [to $0^{3,5}$-bis(4-chlorobenzoyl)-2-deoxy-$\alpha$-D-ribofuranosyl chloride].

m.p. 161 ~ 164 °C
Infrared absorption spectrum (KBr) $cm^{-1}$
3380,2940,1720,1660,1270,1085,1050,840,770
$^1$H-NMR spectrum (200MHz,$D_2O$)
$\delta_{(ppm)}$ = 2.40(2H,m,2′-H)
3.77(2H,m,5′-H)
4.05(1H,m,4′-H)
4.40(1H,m,3′-H)
6.20(2H,m,1′-H,5-H)
8.07(1H,d,J=8Hz,6-H)
Optical rotation $[\alpha]_D^{20}$ + 57° (c=6.3,$H_2O$)

Example 2

6.64 g (26 mmol) of bis(trimethylsilyl)cytosine, 3.23 g (20 mmol) of hexamethyldisilazane and 300 ml of chloroform were introduced into a flask (one litre, three necks flask replaced with nitrogen gas), and to the mixture was added 8.59 g (20 mmol) of $0^{3,5}$-bis(4-chlorobenzoyl)-2-deoxy-α-D-ribofuranosyl chloride dissolved in 300 ml of chloroform, while stirring, to subject to condensation at room temperatures for one hour.

After the reaction was completed, the reaction mixture was treated in the same manner as that in Example 1 to obtain 10.8 g of crude $0^{3',5'}$-bis(4-chlorobenzoyl)-2′-deoxycytidine.

By analyzing this compound with high performance liquid chromatography, the ratio of α-type compound to β-type compound of 8/92 was obtained.

The crude product was purified by the same manner as that of Example 1 to obtain 8.81 g of $0^{3',5'}$-bis-(4-chlorobenzoyl)-2′-deoxy-β-cytidine not containing α-type compound. Yield 87 % [to $0^{3,5}$-bis(4-chlorobenzoyl)-2-deoxy-α-D-ribofuranosyl chloride].

$0^{3',5'}$-bis(4-chlorobenzoyl)-2′-deoxy-β-cytidine was treated with ammonia-containing methanol to eliminate the protecting group (4-chlorobenzoyl group) and then concentrated hydrochloric acid was added to produce crude 2′-deoxy-β-cytidine HCl, and the crude salt was recrystallized from water-methanol to obtain 4.38 g of 2′-deoxy-β-cytidine HCl. Yield 83 % [to $0^{3,5}$-bis(4-chlorobenzoyl)-2-deoxy-α-D-ribofuranosyl chloride].

m.p. 161 ~ 164 °C
Infrared absorption spectrum (KBr) $cm^{-1}$
3380,2940,1720,1660,1270,1085,1050,840,770
$^{1}$H-NMR spectrum (200MHz,$D_2O$)
$\delta_{(ppm)}$ = 2.40(2H,m,2′ -H)
3.77(2H,m,5′ -H)
4.05(1H,m,4′ -H)
4.40(1H,m,3′ -H)
6.20(2H,m,1′ -H,5 -H)
8.07(1H,d,J=8Hz,6-H)
Optical rotation $[\alpha]_D^{20}$ + 57 ° (c=6.3,$H_2O$)

Example 3

11.0 g of crude $0^{3',5'}$-bis(4-chlorobenzoyl)-2′-deoxycytidine was obtained by repeating the same procedure as that of Example 2, except that 1.12 g (12 mmol) of triethylamine was used instead of hexamethyldisilazane.

By analyzing the above crude compound with high performance liquid chromatography, the ratio of α-type compound to β-type compound of 9/91 was obtained.

The crude product was recrystallized by the same manner as that of Example 2 to obtain 8.57 g of $0^{3',5'}$-bis(4-chlorobenzoyl)-2′-deoxy-β-cytidine not containing α-type compound. Yield 85 % [to $0^{3,5}$-bis(4-chlorobenzoyl)-2-deoxy-α-D-ribofuranosyl chloride].

After the protecting group of $0^{3',5'}$-bis(4-chlorobenzoyl)-2′-deoxy-β-cytidine was eliminated by the same manner as that of Example 2, and then a salt of hydrochloric acid was prepared and the salt was recrystallized to obtain 4.24 g of 2′-deoxy-β-cytidine HCl. Yield 80 % [to $0^{3,5}$-bis(4-chlorobenzoyl)-2-deoxy-α-D-ribofuranosyl chloride].

m.p. 161 ~ 164 °C
Infrared absorption spectrum (KBr) $cm^{-1}$
3380,2940,1720,1660,1270,1085,1050,840,770
$^{1}$H-NMR spectrum (200MHz,$D_2O$)
$\delta_{(ppm)}$ = 2.40(2H,m,2′ -H)
3.77(2H,m,5′ -H)
4.05(1H,m,4′ -H)
4.40(1H,m,3′ -H)
6.20(2H,m,1′ -H,5-H)
8.07(1H,d,J=8Hz,6-H)
Optical rotation $[\alpha]_D^{20}$ + 57 ° (c=6.3,$H_2O$)

Comparative Example 1

9.7 g of crude $0^{3',5'}$-bis(4-chlorobenzoyl)-2′-deoxycytidine was obtained by repeating the same procedure as that in Example 1 except that 6.64 g (26 mmol) of bis(trimethylsilyl)cytosine was used instead of 25.5 g (100 mmol) thereof.

By analyzing the above crude product with high performance liquid chromatography, the ratio of $\alpha$-type compound to $\beta$-type compound of 41/59 was obtained.

Example 4

12.7 g of crude $0^{3',5'}$-bis(4-chlorobenzoyl)-$N^4$-benzoyl-2′-deoxycytidine was obtained by repeating the same procedure as that of Example 1, except that 22.9 g (80 mmol) of $N^4$-benzoyl-$0^2$-trimethylsilylcytosine was used instead of bis(trimethylsilyl)cytosine used in Example 1.

By analyzing the above crude product with high performance liquid chromatography, the ratio of $\alpha$-type compound to $\beta$-type compound of 10/90 was obtained.

The above crude product was purified by the same manner as that of the Example 1 to obtain 9.85 g of $0^{3',5'}$-bis (4-chlorobenzoyl)-$N^4$-benzoyl-2′-deoxy-$\beta$-cytidine not containing $\alpha$-type compound. Yield 81 % [to $0^{3,5}$-bis(4-chlorobenzoyl)-2-deoxy-$\alpha$-D-ribofuranosyl chloride].

$0^{3',5'}$-bis(4-chlorobenzoyl)-$N^4$-benzoyl-2′-deoxy-$\beta$-cytidine was treated with ammonia-containing methanol to eliminate the protecting groups (benzoyl group and 4-chlorobenzoyl group), and then concentrated hydrochloric acid was added to produce crude 2′-deoxy-$\beta$-cytidine HCl, and the crude salt was recrystallized from water-methanol to obtain 4.05 g of 2′-deoxy-$\beta$-cytidine HCl. Yield 77 % [to $0^{3,5}$-bis(4-chlorobenzoyl)-2-deoxy-$\alpha$-D-ribofuranosyl chloride].

m.p. 161 ~ 164 °C
Infrared absorption spectrum (KBr) $cm^{-1}$
3380,2940,1720,1660,1270,1085,1050,840,770
$^1$H-NMR spectrum (200MHz,$D_2O$)
$\delta_{(ppm)}$ = 2.40(2H,m,2′ -H)
3.77(2H,m,5′ -H)
4.05(1H,m,4′ -H)
4.40(1H,m,3′ -H)
6.20(2H,m,1′ -H,5-H)
8.07(1H,d,J = 8Hz,6-H)
Optical rotation $[\alpha]_D^{20}$ + 57 ° (c = 6.3,$H_2O$)

Example 5

12.5 g of crude $0^{3',5'}$-bis(4-chlorobenzoyl)-$N^4$-benzoyl-2′-deoxycytidine was obtained by repeating the same procedure as that of Example 2, except that 7.46 g (26 mmol) of $N^4$-benzoyl-$O^2$-trimethylsilylcytosine was used instead of bis(trimethylsilyl)cytosine used in Example 2.

By analyzing the above crude product with high performance liquid chromatography, the ratio of $\alpha$-type compound to $\beta$-type compound of 10/90 was obtained.

The above crude product was purified by the same manner as that of Example 2 to obtain 10.1 g of $0^{3',5'}$-bis(4-chlorobenzoyl)-$N^4$-benzoyl-2′-deoxy-$\beta$-cytidine. Yield 83 % [to $0^{3,5}$-bis(4-chlorobenzoyl)-2-deoxy-$\alpha$-D-ribofuranosyl chloride].

$0^{3',5'}$-bis(4-chlorobenzoyl)-$N^4$-benzoyl-2′-deoxy-$\beta$-cytidine was treated in the same manner as that of Example 4 to eliminate the protecting groups, and a crude salt of hydrochloric acid was produced and then purified to obtain 4.16 g of 2′-deoxy-$\beta$-cytidine HCl. Yield 79 % [to $0^{3,5}$-bis (4-chlorobenzoyl )-2-deoxy-$\alpha$-D-ribofuranosyl chloride].

m.p. 161 ~ 164 °C
Infrared absorption spectrum (KBr) $cm^{-1}$
3380,2940,1720,1660,1270,1085,1050,840,770
$^1$H-NMR spectrum (200MHz,$D_2O$)
$\delta_{(ppm)}$ = 2.40(2H,m,2′ -H)
3.77(2H,m,5′ -H)
4.05(1H,m,4′ -H)
4.40(1H,m,3′ -H)
6.20(2H,m,1′ -H,5-H)

8.07(1H,d,J = 8Hz,6-H)
Optical rotation $[\alpha]_D^{20}$ + 57 ° (c = 6.3,$H_2O$)

Comparative Example 2

12.1 g of crude $0^{3',5'}$-bis(4-chlorobenzoyl)-$N^4$-benzoyl-2′-deoxycytidine was obtained by repeating the same procedure as that of Example 4, except that 7.46 g (26 mmol) of $N^4$-benzoyl-$0^2$-trimethylsilylcytosine was used instead of 22.9 g (80 mmol) thereof.

By analyzing the above crude product with high performance liquid chromatography, the ratio of α-type compound to β-type compound of 53/47 was obtained.

**Claims**

**1.** Process for preparing derivatives of 2'-deoxy-β-cytidine having the following general formula [I]

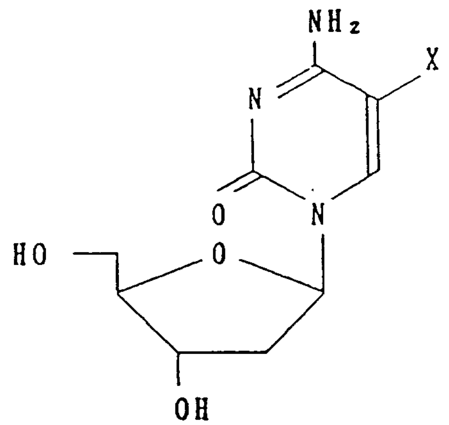

( I )

(wherein X is a hydrogen atom, a halogen atom, or an alkyl group or alkenyl group which may be substituted with one or more halogen atoms) and salts thereof,
characterized in that a derivative of 1-α-halogeno-2-deoxyribose having the following general formula [III]:

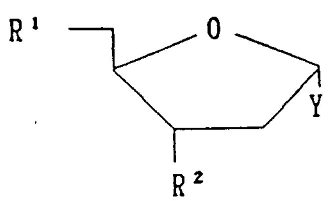

( III )

(wherein $R^1$ and $R^2$ are independently protected hydroxyl groups and Y is a halogen atom)
is allowed to condense with a derivative of cytosine having the following general formula [IV]:

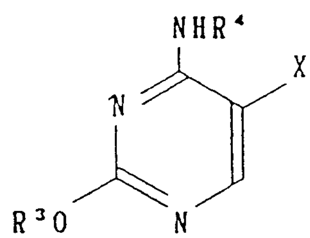

( IV )

(wherein $R^3$ is hydroxyl protecting group, $R^4$ is an amine protecting group and X is as defined above)
in the presence of ammonia or an amine using 1.1 to 1.5 equivalents of cytosine derivative (IV) per

equivalent of ribose derivative (III) and 0.5 to 1.5 equivalents of ammonia or amine per equivalent of cytosine derivative (IV) or in the absence of ammonia or an amine using 3 to 5 equivalents of cytidine derivative (IV) per equivalent of ribose derivative (III) to obtain a derivative of 3',5'di-substituted-2'-deoxy-β-cytidine having the following general formula [II]:

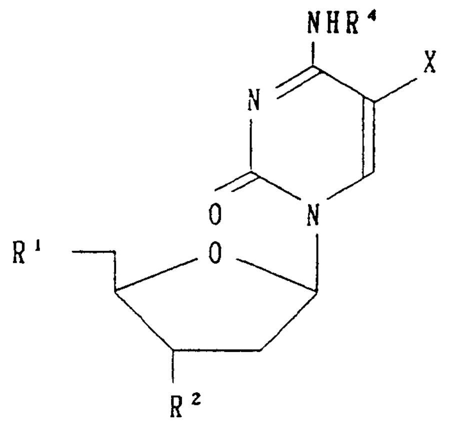

( II )

(wherein $R^1$, $R^2$, $R^4$ and X are as defined above) and then the protecting groups are eliminated.

2. A process as claimed in claim 1 in which the protecting groups in $R^1$ and $R^2$ are aralkyl, acyl, alkoxycarbonyl or aryloxycarbonyl groups.

3. A process as claimed in claim 1 or claim 2 in which the groups $R^3$ and $R^4$ are aralkyl, acyl, alkoxycarbonyl, aryloxycarbonyl or triorganosilyl groups.

4. A process as claimed in any one of claims 1 to 3 in which the condensation reaction is carried out in an aprotic solvent.

**Patentansprüche**

1. Verfahren zur Herstellung von 2'-Desoxy-β-cytidin-Derivaten der nachfolgenden allgemeinen Formel [I]

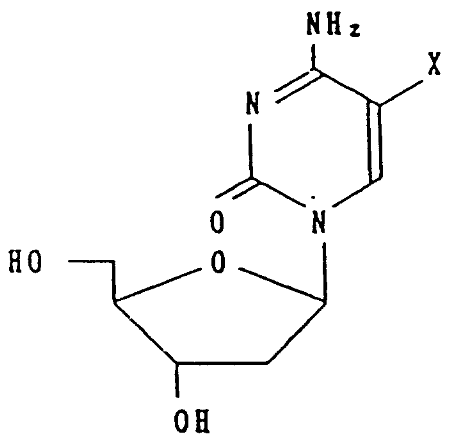

( I )

(worin X ein Wasserstoffatom, ein Halogenatom oder eine Alkylgruppe oder eine Alkenylgruppe ist, die mit einem oder mehreren Halogenatomen substituiert sein kann) und Salzen hiervon,
**dadurch gekennzeichnet**,
daß man ein Derivat von 1-α-Halogeno-2-desoxyribose mit der nachfolgenden allgemeinen Formel [III]:

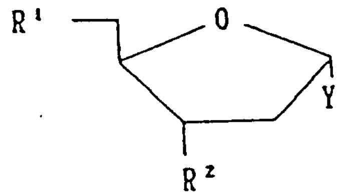

( III )

(worin $R^1$ und $R^2$ unabhängig voneinander geschützte Hydroxylgruppen sind und Y ein Halogenatom ist)
mit einem Cytosin-Derivat der nachfolgenden allgemeinen Formel [IV]:

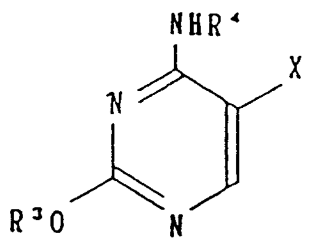

( IV )

(worin $R^3$ eine Hydroxyl-Schutzgruppe, $R^4$ eine Amin-Schutzgruppe und X wie oben definiert ist)
in Gegenwart von Ammoniak oder eines Amins unter Verwendung von 1,1 bis 1,5 Cytosin-Derivat (IV)-Äquivalenten pro Ribose-Derivat (III)-Äquivalent und 0,5 bis 1,5 Ammoniak- oder Amin-Äquivalenten pro Cytosin-Derivat (IV)-Äquivalent oder in Abwesenheit von Ammoniak oder eines Amins unter Verwendung von 3 bis 5 Cytidin-Derivat (IV)-Äquivalenten pro Ribose-Derivat (111)-Äquivalent kondensieren läßt, um ein 3',5'-Di-substituiertes-2'-desoxy-$\beta$-Cytidin-Derivat der nachfolgenden allgemeinen Formel [II] zu erhalten:

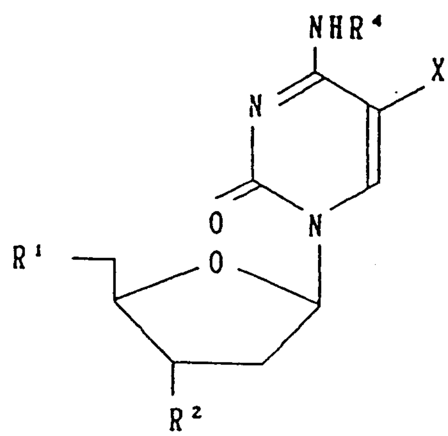

( II )

(worin $R^1$, $R^2$, $R^4$ und X wie oben definiert sind) und dann die Schutzgruppen eliminiert werden.

2. Verfahren nach Anspruch 1, wobei die Schutzgruppen in $R^1$ und $R^2$ Aralkyl-, Acyl-, Alkoxycarbonyl- oder Aryloxycarbonyl-Gruppen sind.

3. Verfahren nach Anspruch 1 oder 2, wobei die Gruppen $R^3$ und $R^4$ Aralkyl-, Acyl-, Alkoxycarbonyl-, Aryloxycarbonyl- oder Triorganosilyl-Gruppen sind.

4. Verfahren nach einem der Ansprüche 1 bis 3, wobei die Kondensationsreaktion in einem aprotischen Lösungsmittel durchgeführt wird.

## Revendications

1. Procédé pour préparer des dérivés de la 2'-désoxy-β-cytidine ayant la formule générale [I] suivante :

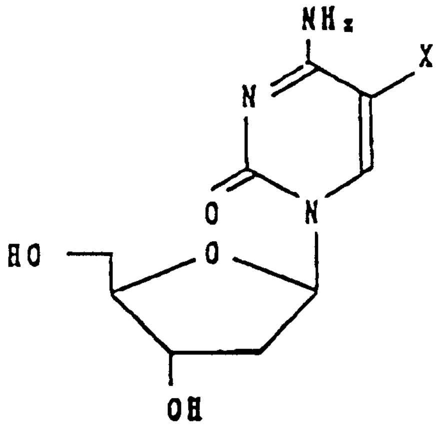

( I )

(dans laquelle X est un atome d'hydrogène, un atome d'halogène ou un groupe alkyle ou alcényle qui peut être substitué avec un ou plusieurs atomes d'halogène)
et leurs sels,
caractérisé en ce qu'un dérivé de 1-α-halogéno-2-désoxyribose ayant la formule générale [III] suivante :

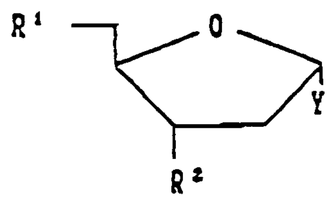

( III )

(dans laquelle $R^1$ et $R^2$ sont indépendamment des groupes hydroxyles protégés et Y est un atome d'halogène)
est amené à se condenser avec un dérivé de la cytosine ayant la formule générale [VI] suivante :

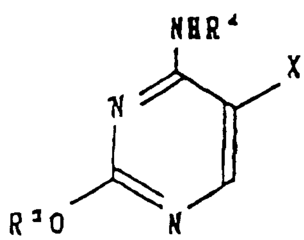

( IV )

(dans laquelle $R^3$ est un groupe protecteur d'hydroxyle, $R^4$ est un groupe protecteur d'amine et X est tel que défini ci-dessus)
eu présence d'ammoniac ou d'une amine à l'aide de 1,1 à 1,5 équivalent de dérivé de la cytosine (IV) par équivalent de dérivé du ribose (III) et de 0,5 à 1,5 équivalent d'ammoniac ou d'amine par équivalent de dérivé de la cytosine (IV) ou en l'absence d,ammoniac ou d'une amine à l'aide de 3 à 5 équivalents de dérivé de la cytosine (IV) par équivalent de dérivé du ribose (III) pour obtenir un dérivé de 2'-désoxy-β-cytidine 3',5'-disubstitué ayant la formule générale [II] suivante :

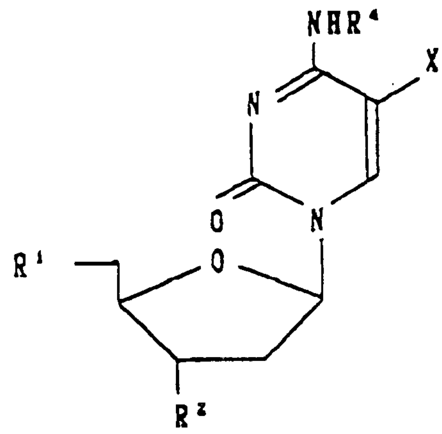

(II)

(dans laquelle $R^1$, $R^2$, $R^4$ et X sont tels que définis ci-dessus) puis les groupes protecteurs sont éliminés.

2. Procédé selon la revendication 1, dans lequel les groupes protecteurs dans $R^1$ et $R^2$ sont des groupes aralkyles, acyles, alcoxycarbonyles ou aryloxycarbonyles.

3. Procédé selon la revendication 1 ou la revendication 2, dans lequel les groupes $R^3$ et $R^4$ sont des groupes aralkyles, acyles, alcoxycarbonyles, aryloxycarbonyles ou triorganosilyles.

4. Procédé selon l'une quelconque des revendications 1 à 3, dans lequel la réaction de condensation est conduite dans un solvant aprotique.